# EUROPEAN PATENT APPLICATION

(11) **EP 4 790 132 A1**
(43) Date of publication of application: **12.08.2026**
(21) Application number: 25382079.9
(22) Date of filing: 05.02.2025
(51) Int. Cl.: C12N 5/071

(54) **METHOD OF PREPARING A CELLULAR MODEL**

(71) Applicant: Provital, S.A., 08210 Barberà del Vallès (ES)
(72) Inventor: BAJSERT, Julia, 08930 Sant Adrià De Besòs (ES); MANZANO ALÍAS, David, 08025 Barcelona (ES); JIMÉNEZ ALTAYÓ, Francesc, 08415 Bigues I Riells (ES); ASO PÉREZ, Miguel, 08191 Rubí (ES)
(74) Representative: Sugrañes, S.L.P.

(57) **Abstract**

The present invention relates to a method of preparing an in vitro or ex vivo cell culture or a cellular model of atopic dermatitis or of pityriasis alba comprising a step of exposing a population of cells comprising keratinocytes and melanocytes to a culture medium, the culture medium comprising cytokines selected from the group consisting of IL-4, IL-13, IL-31 and combinations thereof. The present invention also relates to a cell culture obtained by such method, wherein the cell culture comprises keratinocytes, melanocytes and cytokines selected from the group consisting of IL-4, IL-13, IL-31 and combinations thereof. The present invention also relates to a cellular model of atopic dermatitis or of pityriasis alba obtained by such method, wherein the cellular model comprises keratinocytes and melanocytes. The present invention also relates to the use of such cell culture or of such cellular model for studying in vitro or ex vivo pigmentation/depigmentation mechanisms and modulators of these mechanisms and for screening in vitro or ex vivo pharmaceutically or cosmetically active molecules suitable for preventing, treating or attenuating a skin disorder or conditioning a skin in a subject in need thereof.

## Description

### Technical field of the invention

The present invention relates to the field of tissue engineering, dermatology and cosmetics. The present invention more specifically relates to a method of preparing an in vitro or ex vivo cellular model. It further relates to a cellular model obtained by such method and to a cellular culture. It further relates to uses of the model in experimental research, typically for studying pigmentation/depigmentation mechanisms and for the screening of molecules of interest in the prevention or treatment of skin disorders, in particular atopic dermatitis or pityriasis alba, and in cosmetic applications.

### Background of the invention

The skin is a large and complex tissue providing a protective interface between an organism and its environment. Epidermis forms its external surface and is mainly constituted of multiple layers of specialized epithelial cells named keratinocytes. Melanocytes are cells producing melanin located in the bottom layer of the epidermis and are responsible for the production of melanin: the pigment primarily responsible for skin color. Skin can be injured by many different causes, including microorganisms, chemicals, behaviors, physical injury, ageing, U.V. irradiation, cancer, autoimmune or inflammatory diseases.

The present invention advantageously provides new means for studying and understanding specific mechanisms leading to a disorder of skin pigmentation.

Reconstructed human epidermis (RHE) is defined as a three-dimensional in vitro reconstruction of the human epidermis from primary keratinocytes extracted from human skin. These models are histologically and functionally similar to the human epidermis in vivo. However, it is not possible to reconstruct the actual immune response in RHE models. Induction of an RHE that mimics atopic dermatitis (AD) can be accomplished due to modification of the culture medium for different RHE models. The etiology of AD is complex and not fully understood to date. Both epidermal barrier impairment and systemic inflammation, resulting in an abnormal immune response, lead to a vicious cycle. In fact, epidermal barrier defects allow an increase in cutaneous permeability and consequently trigger inflammation and activation of the immune system (the "outside-in" hypothesis). Conversely, skin inflammation leads to epidermal barrier defects with disruption of keratinocyte differentiation (the "inside-out" hypothesis). The acute inflammatory phase begins with a predominant T helper 2 (Th2) lymphocyte response and then evolves into a chronic T helper 1 (Th1) lymphocyte response. In addition, AD skin lesions are characterized by overexpression of proinflammatory type 2 molecules such as interleukin-4 (IL-4), IL-13 and thymic stromal lymphopoietin (TSLP) secreted by keratinocytes. Inflammation is also maintained, enhanced or triggered by neurogenic inflammation. As for the epidermis, both the organization and composition of proteins and lipids play an important role in epidermal barrier function. RHE models are valuable tools to study specific aspects of epidermal pathophysiology, especially for AD. However, the absence of fibroblasts, immune cells and nerve endings remains one of the main problems of the RHE model.

Skin barrier defects are very common and although most conditions are benign, certain diseases such as atopic dermatitis (AD) can have significant debilitating effects coupled with inflammation. Although great progress has been made in understanding the pathogenesis of AD, barrier dysfunction linked to a complex inflammatory environment remains poorly understood. Current therapies generally aim to decrease cutaneous inflammation and alleviate pruritus but fail to correct the underlying barrier defects that drive the process. To meet this need, intensive research is underway to develop specific strategies to restore epidermal integrity. In this search for new treatments, the need for efficient in vitro assays has become crucial.

RHEs facilitate the investigation of specific cellular responses of keratinocytes and can help to estimate the influence of different inflammatory cytokines. They are considerably less complex than in vivo settings, and intensified research efforts are needed to further mimic in vitro disease patterns and allow the study of pathophysiological aspects in a more complex environment. However, increasing the complexity of models will necessarily accentuate other problems, such as lack of comparability, and may produce contradictory results. In the context of drug screening and mechanistic investigations, RHE models benefit from a certain balance between complexity, standardization, cost, and biological relevance. By using multiple specific models, it is possible to target antiinflammatory properties with one model, while another focuses on identifying active substances that can restore epidermal homeostasis and barrier function in an inflammatory environment.

The key point today is to validate these models by presenting appropriate controls. In vitro skin models range from simple RHE to equivalent whole skin complexes integrating immune cells or microfluidic platforms. Complexity is relevant when studying cell type interactions, in particular interactions with the immune system. However, complex models may present biological variations and higher production costs. Thus, depending on research needs, RHEs offer a good compromise between complexity and biological relevance. Therefore, standardized and reproducible RHE are relevant models for therapeutic research evaluating new compounds targeting epidermal features in these models.

For instance, EP3336175A1 discloses a three-dimensional cellular model of depigmenting disorders as well as to products and methods for preparing such a model, and uses of the model in experimental research, typically for studying depigmentation mechanisms and for the screening of molecules of interest in the prevention or treatment of pathological conditions wherein a depigmenting disorder is observed, in particular vitiligo as well as other inflammatory dermatoses.

It is known that in atopic dermatitis, Th2 cells secrete IL-4, IL-13 and IL-31 acting as major drivers of barrier defects by reducing differentiation markers such as filaggrin, loricrin and involucrin.

Although significant advances have been made in understanding the pathogenesis of AD, many aspects remain poorly understood. In addition, there is a pressing need for improved therapeutic options. In recent decades, studies to elucidate the pathophysiological pathways of AD and identify new therapeutic targets have been conducted almost exclusively in animal models. However, in vitro approaches, such as 3D models of skin diseases, have recently emerged due to an increased awareness of distinct species-related differences that make it difficult to effectively translate results from animal models to humans. In addition, there is increasing political and societal pressure to develop alternatives to animal models in accordance with the 3R principle (reduction, refinement, and replacement of animal models).

Over the years, several models of AD have been developed. Some have used patient cells to understand the respective roles of fibroblasts and keratinocytes in the disease. Others have used Th2 cytokines, such as IL-4 and IL-13, to reproduce the AD phenotype. Genomic approaches, including filaggrin knockout or knockdown, have been investigated.

For instance, EP3027731A2 relates to the use of a pro-inflammatory stimulating reagent containing between 10 and 500 ng/mL of interleukin IL-1β; to reproduce the physiopathology of atopic dermatitis in vitro and/or to alter the barrier function in a cutaneous substitute or a cutaneous explant.

Pityriasis alba (PA) is a common and benign skin condition primarily affecting children and teenagers of all races. This dermatological disorder observed in up to 40% of dark-skinned children and around 2% of light-skinned children occurs with a similar prevalence in both sexes. PA is manifested by hypopigmented, superficial, off-white to light tan scaling macules that are mainly confined to the face but can also appear on other areas of the body such as arms or shoulders. PA tends to be more apparent in individuals with darker skin types. Even though the color alterations in the skin are self-resolving and can last up to few years, these visible pigmentary changes on the skin can often lead to diminished self-esteem and significantly lowered patient's quality of life.

PA is regarded as a type of post-inflammatory hypopigmentation (PIH) disorder, stemming from an inflammatory pathology. There is a strong correlation between PA and atopic dermatitis (AD), as repeatedly reported. Additionally, it has been demonstrated that individuals with a history of atopy are at greater risk of developing PA. AD is the most frequent chronic inflammatory skin disorder and is characterized by an immunological imbalance that results in a predominance of T helper type 2 (Th2) cells, with Th2 cytokines, interleukin-4 (IL-4), IL-13 and IL-31, playing a central role in the development and progression of this inflammatory disease.

Although PA is a widespread pigmentary disorder, little effort has been made to better comprehend the mechanism behind this pathology. As a result, etiopathology and histological changes in PA remain unclear. Although a decrease in melanin pigment and spongiotic edema in PA is well described, there are conflicting findings in the scientific literature when studying the morphology and functionality of melanocytes. Some studies performed on skin biopsies of patients with PA showed a reduction in the quantity of functional melanocytes together with a reduction of the size and number of melanosomes. Other researchers described no alteration in the quantity of melanocytes, but reduced number of melanosomes. On the other hand, while there has been described no impairment of melanosome transfer in patients with PA, others have found an alteration when transferring melanosomes to keratinocytes. The complexity of the etiology of PA-associated hypomelanosis, combined with the lack of consensus on the underlaying mechanisms, has prompted the applicant to develop a novel in vitro pigmented skin model of PA to gain a deeper understanding of this pigmentary disorder.

### Summary of the invention

In a first aspect, the present invention relates to a method of preparing an in vitro or ex vivo cell culture or a cellular model of atopic dermatitis or of pityriasis alba comprising a step of exposing a population of cells comprising keratinocytes and melanocytes to a culture medium, the culture medium comprising cytokines selected from the group consisting of IL-4, IL-13, IL-31 and combinations thereof.

In a second aspect, the present invention relates to a cell culture obtained by the method described herein, wherein the cell culture comprises keratinocytes, melanocytes and cytokines selected from the group consisting of IL-4, IL-13, IL-31 and combinations thereof.

In a third aspect, the present invention relates to a cellular model of atopic dermatitis or of pityriasis alba obtained by the method described herein, wherein the cellular model comprises keratinocytes and melanocytes.

In a fourth aspect, the present invention relates to a use of the cell culture described herein or of the cellular model described herein for studying in vitro or ex vivo pigmentation/depigmentation mechanisms and modulators of these mechanisms.

In a fifth aspect, the present invention relates to a use of the cell culture described herein or of the cellular model described herein in a method for screening in vitro or ex vivo pharmaceutically active molecules suitable for preventing, treating or attenuating a skin disorder in a subject in need thereof.

In a sixth aspect, the present invention relates to a use of the cell culture described herein or of the cellular model described herein in a method for screening in vitro or ex vivo cosmetically active ingredients suitable for a conditioning effect on the skin in a subject in need thereof.

In a seventh aspect, the present invention relates to a use of a composition containing a combination of cytokines selected from the group consisting of IL-4, IL-13, IL-31 and combinations thereof for reproducing in vitro or ex vivo the pathophysiology of atopic dermatitis or pityriasis alba in reconstructed skin or reconstructed epidermis.

Other features of the invention are disclosed in the detailed description of the invention.

### Brief description of the figures

The foregoing and other advantages and features will be more fully understood from the following detailed description of the invention with reference to the accompanying figures to be taken in an illustrative and non-limitative manner, in which:

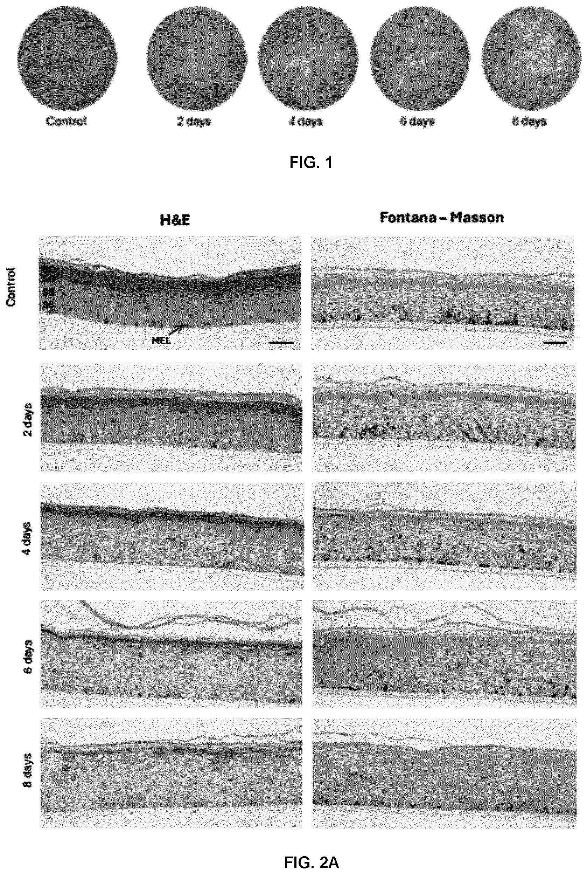

FIG. 1 shows melanin changes in reconstructed human pigmented epidermis (RHPE) upon stimulation of the cytokines as disclosed in the present invention. FIG. 1 corresponds to magnified pictures (8x) of reconstructed human pigmented epidermis captured with stereomicroscope at day 17 after stimulation of cytokines during 2, 4, 6 or 8 days and compared to unstimulated control.

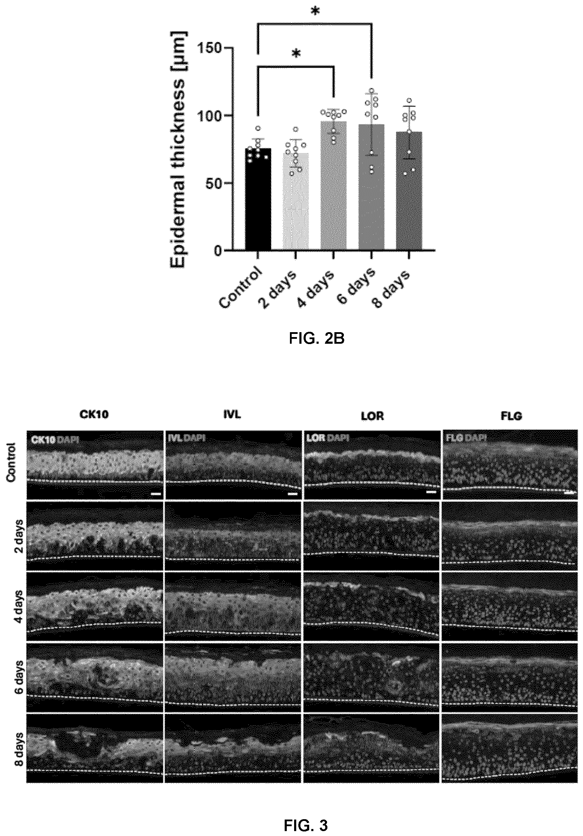

FIG. 2 shows morphological alterations in RHPE after treatment with the cytokines as disclosed in the present invention. FIG. 2A corresponds to Hematoxylin and Eosin (H&E) and Fontana-Masson staining of epidermis equivalents stimulated with cytokines mix for 2, 4, 6 or 8 days and compared to an unstimulated control. MEL, melanocyte; SB, *stratum basale;* SC, *stratum corneum;* SG, *stratum granulosum;* SS, *stratum spinosum.* Scale bar: 50 µm; pictures are representatives of n=3 independent experiments with a total of 9 skin models. Magnification 200x. FIG. 2B shows the quantification of the epidermal thickness of RHPE stimulated with cytokines during 2, 4, 6 or 8 days compared to unstimulated control. Viable cellular layer measurements were performed on 9 skin models (two pictures per condition were captured and three measurements of the thickness were taken per picture). Quantification of epidermal thickness (mean + SD) of n=3 independent experiments of a total of 9 skin models. Statistical difference was determined by a one-way ANOVA with Dunett's post hoc test. * P < 0.05 compared to control.
FIG. 3 shows the expression of epidermal differentiation markers in RHPE treated with the cytokines as disclosed in the present invention. Expression of cytokeratin 10 (CK10), involucrin (IVL), loricrin (LOR), filaggrin (FLG) in epidermis equivalents treated with cytokines for 2, 4, 6 or 8 days was investigated by immunofluorescence and compared to an untreated control. Cell nuclei were stained in blue with 4',6-diamidino-2-phenylindoline (DAPI). The dashed line indicates the polycarbonate membrane. Scale bare: 50 µm; pictures are representative of n=3 independent experiments with a total of 9 skin models. Magnification 200x.

### Detailed description of the invention

In one aspect, the present invention discloses a method of preparing an in vitro or ex vivo cell culture or a cellular model of atopic dermatitis or of pityriasis alba comprising a step of exposing a population of cells comprising keratinocytes and melanocytes to a culture medium, the culture medium comprising cytokines selected from the group consisting of IL-4, IL-13, IL-31 and combinations thereof, as defined herein.

The inventors have found that exposing a combination of keratinocytes and melanocytes to a culture medium comprising the at least one of the cytokines selected from the group IL-4, IL-13, IL-31 and combinations thereof, a model having the phenotype of atopic dermatitis or pityriasis alba is obtained.

In particular, the cytokines present in the medium may be in a concentration preferably ranging from 1 to 50 ng/mL, preferably from 1 to 40 ng/mL, preferably from 5 to 40 ng/mL, preferably from 10 to 40 ng/mL, preferably from 20 to 40 ng/mL, preferably about 30 ng/mL. Cytokines are diluted in the medium which is typically a culture medium or a culture medium as herein described.

The cytokines selected may preferably be a combination of the three cytokines IL-4, IL-13 and IL-31. The cytokines may be selected from a combination of IL-4 and IL-13, a combination of IL-4 and IL-31 and a combination of IL-13 and IL-31. Preferably, the cytokines may be IL-4, IL-13 and IL-31.

The culture medium may further comprise other components such as polyinosinic:polycytidylic acid, methyl β-cyclodextrin, T-cells, bacterial colonies and/or other cytokines different from IL-4, IL-13 and IL-31, the cytokines being selected from the group consisting of IL-22, IL-25, IL-17, TNFα and combinations thereof.

In the method according to the invention, the cells are preferably human cells, even more preferably from a healthy subject having dark skin.

By dark skin, the skilled in the art understands a type of human skin color that is rich in melanin pigments and have melanosomes which are clumped, large organelles full of eumelanin.

In the method according to the invention, the ratio of keratinocytes and melanocytes is between 1:1 and 100:1, preferably between 1:1 and 50:1, more preferably between 10:1 and 50:1, more preferably between 10:1 and 30:1, even more preferably about 20:1.

The method according to the invention may preferably comprise a step of culturing the cells at an air-liquid interface.

An air-liquid interface is understood in the art as a method of cell culture by which basal stem cells are grown with their basal surfaces in contact with culture media, and the top of the cellular layer is exposed to the air.

In a preferred embodiment, the in vitro or ex vivo cellular model is a reconstructed skin model, preferably a reconstructed human skin model, more preferably a three-dimensional reconstructed skin model, even more preferably a three-dimensional reconstructed human skin model.

Even more preferably, the in vitro or ex vivo cellular model is a reconstructed human pigmented epidermis (RHPE) model, or even more preferably a three-dimensional reconstructed human pigmented epidermis (RHPE) model.

In the context of the present invention, reconstructed human pigmented epidermis (RHPE) refers to a laboratory-grown model of human skin that mimics the structure and function of the epidermis, particularly its pigmentation. This model is typically created by culturing human epidermal cells, such as keratinocytes, in a controlled environment, and incorporates melanocytes, the cells responsible for producing melanin, which gives skin its color.

In RHPE, three key features of the human skin are replicated, including stratification, where skin cells develop into layers, pigmentation, where varying skin tones are generated in the model, and barrier function, enabling the model to be a protective barrier against environmental stressors.

Cells are typically exposed to cytokines for a period varying between about one hour and 6, 12, 24, 48, 72, 96, 120, 144, 168, 192, 216 or 240 hours, preferably 48, 96, 144 or 192 hours. Preferably, cells are exposed to cytokines for 48 and 96 hours.

In another aspect, the present invention also discloses a cell culture obtained by a method described hereinabove, wherein the cell culture comprises keratinocytes, melanocytes and cytokines selected from the group consisting of IL-4, IL-13, IL-31 and combinations thereof.

In a further aspect, the present invention discloses a cellular model of atopic dermatitis or of pityriasis alba obtained by the method disclosed herein, wherein the cellular model comprises keratinocytes and melanocytes.

A typical cellular model described by inventors is an in vitro or ex vivo three-dimensional cellular model. Such a cellular model is typically a skin model, an epidermis model, or a tissue model comprising an epidermis and a dermis. The cellular model might be a reconstructed human pigmented skin, or a reconstructed human pigmented epidermis.

The herein described cellular model is typically prepared from mammal cells, for example from non-human primate (monkey) cells, from domesticated animal cells (e.g., cow, sheep, pig, rabbit, cat, dog, and horse cells), or from rodent cells (such as rat or mouse cells). The cellular model is preferably prepared from human cells.

In the context of the invention, the "subject" is an animal, typically a mammal. Examples of mammals include humans and non-human animals such as, without limitation, domesticated animals (e.g., cows, sheeps, pigs, rabbits, cats, dogs, and horses), non-human primates (such as monkeys), and rodents (e.g., mice and rats). The "subject" is preferably a human being, whatever its gender, age, race or sex.

In such a context, the cells used to prepare the model are preferably healthy cells from the donor in other words cells from a biopsy performed in an unaffected area of the patient, typically a pigmented area of the skin donor.

The cells obtained from the skin donor might be cultured in standard culture medium classically used by the skilled person. Alternatively, commercially available cells might be used instead. The standard culture for keratinocytes might be cultured keratinocyte growth medium 2 (KBM2; #C-20011; PromoCell), while melanocytes might be cultured in melanocyte growth medium 2 (MGM2; #C-24300; PromoCell). Both keratinocytes and melanocytes might typically be grown up to 80% of confluence.

The population of cells can be used to prepare a reconstructed cellular structure such as a skin, an epidermis or a tissue-like or skin-like structure containing an epidermis and a dermis. Such a population of cells or reconstructed cellular/cell structure is typically exposed to a culture medium.

Culture medium usable in the context of the present invention are standard culture medium classically used by the skilled person such as Epilife^{®} Medium (Thermo Fisher Scientific, Waltham, MA, USA), supplemented with human keratinocyte growth supplement (Thermo Fisher Scientific, Waltham, MA, USA).

A culture medium usable in the context of the invention is for example a coculture combining EpiLife^{®} Medium, such as for example the base medium reference MEPI500CA, and human keratinocyte growth supplement, such as HKGS S0015.

The culture medium might be typically supplemented for example with vitamin C, gentamicin, keratinocyte growth factor (KGF) and/or CaCl₂.

Preferably, the (co)culture medium is supplemented with 50 µg/ml of vitamin C, 50 µg/ml of gentamicin, 10 ng/ml of keratinocyte growth factor (KGF) and 1,5 mM of CaCl₂.

Preferably, the (co)culture medium is incubated at 37°C, 5% CO₂.

During air liquid interface cultivation, the models are preferably stimulated every two days by the cytokines so that all epidermis equivalents are air-lifted for 14 days at 37°C under 5% CO₂ and harvested at that same day.

In a further aspect, the present invention discloses a use of a cell culture as disclosed herein or of a cellular model as disclosed herein for studying in vitro or ex vivo pigmentation/depigmentation mechanisms and modulators of these mechanisms.

In particular, that use may be related to a pharmaceutically related method, that is, a method for screening in vitro or ex vivo pharmaceutically active molecules suitable for preventing, treating or attenuating a skin disorder in a subject in need thereof. The skin disorder may preferably be atopic dermatitis or pityriasis alba.

Alternatively, that use may be related to a cosmetically related method, that is, a method for screening in vitro or ex vivo cosmetically active ingredients suitable for a conditioning effect on the skin in a subject in need thereof.

In a further aspect, the present invention also discloses a use of a composition containing a combination of cytokines selected from the group consisting of IL-4, IL-13, IL-31 and combinations thereof for reproducing in vitro or ex vivo the pathophysiology of atopic dermatitis or pityriasis alba in reconstructed skin or reconstructed epidermis.

The group consisting of IL-4, IL-13, IL-31 and combinations thereof may further comprise other components such as polyinosinic:polycytidylic acid, methyl β-cyclodextrin, T-cells, bacterial colonies and/or other cytokines different from IL-4, IL-13 and IL-31, the cytokines being selected from the group consisting of IL-22, IL-25, IL-17, TNFα and combinations thereof.

Therefore, the proposed cell culture and cellular model proposed is generated from human keratinocyte and melanocyte cell lines from donors, preferably with dark skin. Preferably, the model is three-dimensional and contains four layers: stratum corneum, stratum spinosum, granulosum and basale. The phenotype of atopic dermatitis is induced by introducing a cytokine cocktail into the culture (at least a mixture consisting of the group selected from cytokines IL-4, IL-13 and IL-31). As a result, the macroscopic patterns of variable pigmentation that occur in pityriasis alba can be observed in the model and much similarity to the phenotype of the PA patients is achieved.

A typical feature of the model herein disclosed relates to melanin distribution upon stimulation with the cytokine cocktail being strongly impaired in a timely fashion, with the formation of both hypopigmented areas and hyperpigmented spots, similar to the clinical manifestation of melanin distortion found in PA patients.

Another typical feature of the model herein disclosed relates to the obtention of a fully differentiated epidermis with the basal layer (*stratum basale,* SB) containing mostly melanocytes and columnar keratinocytes, the spinous layer (*stratum spinosum,* SS) consisting of polyhedral keratinocytes which are connected to basal and other spinous cells by desmosomes, keratohyalin granules being localized in the granular layer (*stratum granulosum,* SG) and several layers of cornified strata (*stratum corneum,* SC) thereby physiologically reproducing every layer of the human epidermis.

Another typical feature of the model herein disclosed relates to the expression of four key structural proteins, those proteins being cytokeratin 10 (CK10), involucrin (IVL), loricrin (LOR), and filaggrin (FLG). In particular, the model accounts for the reduction of the total expression of CK10, with a diminished distribution in the lower layers of the reconstructed epidermis; the increase in IVL expression being prominently present in the lower layers, including the *stratum basale* (SB), but not being detectable in the SB layer at day 6 and by day 8 having its total content markedly decreased; the finding of LOR in the lower layers of the epidermis, extending to the *stratum spinosum,* although its total expression being consistently reduced; and a no clear change in the total content of distribution of FLG expression.

### Example 1: Cell culture

Normal human epidermal keratinocytes (HEK; #C-12001; PromoCell, Heidelberg, Germany) were maintained in keratinocyte growth medium 2 (KBM2; #C-20011; PromoCell) and cultured until reaching 80% of confluency. Normal human epidermal melanocytes from a darkly pigmented donor (HEM-DP; #C2025C, Thermo Fisher Scientific, Waltham, MA, USA) were cultured in melanocyte growth medium 2 (MGM2; #C-24300; PromoCell) up to 80% of confluence. Cells were maintained at 37 °C with 5% CO₂ under a humified atmosphere.

### Example 2: Obtention of reconstructed human pigmented epidermis (RHPE) model and induction of AD and PA

For the RHPE reconstruction, HEM-DP sat passage 4 and HEK at passage 5 were seeded at a combined density of 500.000 cells/cm² onto a 0.47 cm² insert with polycarbonate membrane (Thermo Fisher Scientific, Waltham, MA, USA) in a ratio of 1:20, respectively. Then, inserts were placed in 24-well plates containing 1.5 mL of coculture medium combining 95% of EpiLife medium (#MEPI500CA; Thermo Fisher Scientific) with human keratinocyte growth supplement (HKGS; #S0015; Thermo Fisher Scientific), 1.5 mM CaCh and 5% of MGM2. After 72 h of incubation at 37°C, 5% CO₂, seeded cells were exposed to an air-liquid interface (ALI), and the medium was then replaced by coculture medium additionally supplemented with 10 ng/ml keratinocyte growth factor (KGF; Sigma Aldrich, St. Louis, MO, USA), 50 µg/ml of vitamin C (Sigma Aldrich), and 50 µg/ml of gentamicin (Sigma Aldrich) and incubated at 37°C, 5% CO₂. During ALI cultivation, RHPEs models were stimulated the last 2, 4, 6 or 8 days of each culture with a mix of interleukins consisting of 30 ng/mL IL-4 (#204-IL; R&D Systems, Minneapolis, MN, USA), 30 ng/mL IL-13 (#213-ILB, R&D Systems) and 15 ng/mL IL-31 (#2824-IL, R&D Systems) so that all epidermis equivalents were air-lifted for 14 days at 37°C under 5% CO₂ and harvested at the same day. Stereomicroscopic pictures at low magnifications were captured using stereomicroscope EZ4 W (Leica, Wetzlar, Germany).

To delineate the impact of AD-induction on the pigmentation in human epidermis, RHPE were stimulated for 2, 4, 6, and 8 days with the cytokine cocktail composed by IL-4, IL-13 and IL-31. FIG. 1 shows that melanin distribution in RHPE upon stimulation with the AD-cocktail is strongly impaired in a timely-fashion, with the formation of both hypopigmented areas and hyperpigmented spots, similar to the clinical manifestation of melanin distortion found in PA patients.

### Example 3: Histological staining of RHPE

To investigate the morphology of the reconstructed skin models, H&E staining was carried out in the same samples described above. Samples were processed by being fixed for 24 h with 4% paraformaldehyde and then dehydrated with progressively increasing concentration of ethanol to remove water, followed by two baths of xylene to eliminate alcohol before paraffin embedding. Later, 5 µm-thick cross sections of paraffin-embedded specimens were placed onto glass slides (VWR, Mississauga, ON, USA). Samples were deparaffinized and rehydrated with three washes of xylene and decreasing ethanol concentration and water. Subsequently, hematoxylin & eosin (H&E) staining was performed. Samples were stained with Harris's hematoxylin (VWR, Mississauga, ON, USA) and washed with tap water. Later, specimens were differentiated with 1% acid ethanol and rinsed with tap water. Then, samples were stained with eosin Y (Sigma Aldrich, St Louis, MO, USA). Next, slides were dehydrated with increasing ethanol series and 3 washes of xylene and mounted with DPX (Sigma Aldrich, St Louis, MO, USA). Fontana-Masson staining was performed using a commercially available kit (#ab150669; abcam, Cambridge, UK), following the manufacturer's instructions. Images were taken with a DM11 microscope (Leica, Wetzlar, Germany) combined with Flexacam C3 camara (Leica, Wetzlar, Germany).

Control condition showed a fully differentiated epidermis with the basal layer (*stratum basale,* SB) containing mostly melanocytes and columnar keratinocytes, as expected from previous reports and from human skin explants. The spinous layer (*stratum spinosum,* SS) consisted of polyhedral keratinocytes which are connected to basal and other spinous cells by desmosomes. Keratohyalin granules are localized in the granular layer (*stratum granulosum,* SG) and several layers of cornified strata (*stratum corneum,* SC) are present, showing that the structure of the model disclosed in the present invention physiologically reproduces every layer of the human epidermis. With regards to pigmentation, the present model shows a melanin content and distribution expected from human epidermis, i.e. melanin accumulation in the basal layer, where melanocytes are located, and an upward distribution of melanin to the upper layers of the epidermis where melanin is more dispersed, as expected from its transfer to neighboring keratinocytes. Moreover, formation of supranuclear caps, accumulations of melanin pigments around the upper side of the nucleus to prevent UV damage to the DNA are present. Taken together, these findings strongly suggest that the pigmentation of the present model closely resembles melanin distribution in human skin.

During stimulation with cytokines to induce AD, remarkable morphological changes were noted following a time-course. Induction of inflammation on the granular layer (SG) results in its gradual thinning. Structural alteration in basal keratinocytes within the *stratum basale* (SB) can be observed, as its shape becomes less cylindrical and more rounded starting at day 2, a trend that increases up to day 8. Moreover, spongiosis is observed in stimulated epidermis equivalents. Furthermore, examining the thickness of the epidermal layers reveals that inflammation also impacts the total epidermal thickness, so that it becomes significantly thicker after 4 and 6 days (FIG. 2B).

To specifically analyze melanin distribution, samples were also stained with Fontana-Masson. In this regard, in the right panel of FIG. 2A it can be observed, again, that the Control samples replicate the melanin distribution found in human skin biopsies, i.e. most of the melanin is distributed in the basal layer, where melanocytes are placed, whereas there is an upwards distribution of melanin pigmentation to upper layers of the epidermis where melanin is much dispersed, and the presence of dendritic-like structures is dramatically reduced with respect to the control, strongly suggesting that the epidermal melanin unit (EMU), composed by one melanocyte distributing melanin to neighboring keratinocytes, is not fully functional. Interestingly, the number of nuclear caps of melanin is also dramatically reduced by AD induction. Similarly, the melanin distribution impairment is accentuated in a timely fashion: melanin is less concentrated in the basal layer and is prevented from entering the upper layers of the epidermis.

### Example 4: Immunohistological staining of RHPE and expression and distribution of key structural proteins therein

5 µm-thick cross sections of paraffin-embedded skin models were placed onto glass slides (Thermo Fisher Scientific, Waltham, MA, USA). Samples were deparaffinized and rehydrated as described above for histological staining. For cytokeratin 14 (CK14), cytokeratin 10 (CK10), filaggrin (FLG) detection, heat mediated antigen retrieval at 95°C with sodium citrate buffer (pH 6.0) was performed. Subsequently, the slides were rinsed with 0.1 M glycine buffer and then incubated for 1 h in PBS containing 0.2% BSA. Afterwards, samples were incubated overnight at 4°C with primary antibodies anti-cytokeratin 14 (1:200, #ab7800, mouse, monoclonal, IgG3, abcam;), anti-cytokeratin 10 (1:200, #ab76318, rabbit, monoclonal, IgG; abcam), anti-filaggrin (1:100, #ab218395, mouse, monoclonal, IgG1; abcam) and anti-Ki67 (1:150, #ab16667, rabbit, monoclonal, IgG; abcam). For involucrin (IVL) and loricrin (LOR) detection, sections were washed with 0.1 M glycine buffer followed by 1 h incubation with PBS containing 0.2% BSA and 0.02% Triton X-100. Then, samples were incubated with primary antibody anti-involucrin (1:200, #I9018, mouse, monoclonal, IgG1; Sigma Aldrich) and anti-loricrin (1:200, #ab85679, rabbit, polyclonal, IgG, abcam, Cambridge, UK) for 1 h. As a control for non-specific labeling, the primary antibody was omitted. For immunofluorescence, samples were incubated with the secondary antibodies anti-rabbit Alexa Fluor 488 (1:200, #A21206, donkey, polyclonal, IgG; Thermo Fisher Scientific) or anti-mouse Alexa Fluor 594 (1:200, #A21203, donkey, polyclonal, IgG; Thermo Fisher Scientific) for 45 min. Slides were mounted with Fluoromount G^{®} mounting medium with DAPI (SouthernBiotech, Birmingham, AL, USA). Images were taken with the fluorescence microscope Eclipse 90i (Nikon, Tokio, Japan). For immunohistochemistry, samples were incubated with secondary antibody anti-rabbit Vectastain ABC HRP kit (1:200, #PK4001, goat, polyclonal, IgG; Vector Laboratories, Newark, CA, USA). Development was performed with the DAB Substrate Chromogen System (Dako, Glostrup, Denmark). Samples were counterstained with hematoxylin for 10 sec and dehydrated as described for histological staining and were mounted with DPX. Images were taken with a DMI1 microscope (Leica) combined with Flexacam C3 camara (Leica).

The impact of the AD-cocktail on four key structural proteins of the human epidermis was assessed, the four key structural proteins being cytokeratin 10 (CK10), involucrin (IVL), loricrin (LOR), and filaggrin (FLG). Fluorescent Immunolabeling of these proteins was performed in the RHPE models according to the present invention (FIG. 3). Upon stimulation with IL-4, IL-13, and IL-31, total expression of CK10 was reduced, with a diminished distribution in the lower layers of the reconstructed epidermis, compared to the Control, where CK10 displayed typical physiological immunolabeling directly above the columnar keratinocytes of the basal layer. The expression and tissue distribution of IVL were markedly altered following cocktail incubation. After 2 and 4 days of treatment, IVL expression increased and was prominently present in the lower layers, including the *stratum basale* (SB), unlike the Control conditions. However, by day 6, IVL was no longer detectable in the SB layer, and by day 8, its total content had markedly decreased. LOR, when stimulated with the AD-cocktail, was found in lower layers of the epidermis, extending to the stratum spinosum, although its total expression was consistently reduced. In contrast, FLG expression did not exhibit a clear change in either total content or distribution.

In the present invention, statistical differences were determined using a one-way ANOVA test followed by Dunett's post hoc test or Kruskal-Wallis test followed by Dunn's test and carried out using GraphPad software (v 10.1.2, GraphPad Software, Inc). A P value of < 0.05 was considered significant.

## Claims

1. A method of preparing an in vitro or ex vivo cell culture or a cellular model of atopic dermatitis or of pityriasis alba comprising a step of exposing a population of cells comprising keratinocytes and melanocytes to a culture medium, the culture medium comprising cytokines selected from the group consisting of IL-4, IL-13, IL-31 and combinations thereof.

2. The method according to claim 1, wherein the cytokines are present in a concentration ranging from 1 to 50 ng/mL of the culture medium.

3. The method according to claims 1 or 2, wherein the cytokines are a combination of IL-4, IL-13 and IL-31.

4. The method according to any one of the previous claims, wherein the culture medium further comprises polyinosinic:polycytidylic acid, methyl β-cyclodextrin and/or cytokines, the cytokines being selected from the group consisting of IL-22, IL-25, IL-17, TNFα and combinations thereof and/or the culture medium further comprises T-cells and/or bacterial colonies.

5. The method according to any one of the previous claims, wherein cells are human cells, and preferably the cells are from a healthy subject having dark skin.

6. The method according to any one of the previous claims, wherein the ratio of keratinocytes and melanocytes is between 1:1 and 100:1.

7. The method according to any one of the previous claims, further comprising a step of culturing the cells at an air-liquid interface.

8. The method according to any one of the previous claims, wherein the in vitro or ex vivo cellular model is a reconstructed skin model.

9. A cell culture obtained by a method according to any one of claims 1 to 8, wherein the cell culture comprises keratinocytes, melanocytes and cytokines selected from the group consisting of IL-4, IL-13, IL-31 and combinations thereof.

10. A cellular model of atopic dermatitis or of pityriasis alba obtained by a method according to any one of claims 1 to 8, wherein the cellular model comprises keratinocytes and melanocytes.

11. Use of a cell culture according to claim 9 or of a cellular model according to claim 10 for studying in vitro or ex vivo pigmentation/depigmentation mechanisms and modulators of these mechanisms.

12. Use of a cell culture according to claim 9 or of a cellular model according to claim 10 in a method for screening in vitro or ex vivo pharmaceutically active molecules suitable for preventing, treating or attenuating a skin disorder in a subject in need thereof.

13. The use according to claim 12, wherein the skin disorder is atopic dermatitis or pityriasis alba.

14. Use of a cell culture according to claim 9 or of a cellular model according to claim 10 in a method for screening in vitro or ex vivo cosmetically active ingredients suitable for a conditioning effect on the skin in a subject in need thereof.

15. Use of a composition containing a combination of cytokines selected from the group consisting of IL-4, IL-13, IL-31 and combinations thereof for reproducing in vitro or ex vivo the pathophysiology of atopic dermatitis or pityriasis alba in reconstructed skin or reconstructed epidermis.
